# EUROPEAN PATENT SPECIFICATION

(11) **EP 1 086 942 B1**
(45) Date of publication and mention of the grant of the patent: **23.04.2003**
(21) Application number: 00119371.3
(22) Date of filing: 11.09.2000
(51) Int. Cl.: C07C 43/196, C07C 69/013, C07C 67/02, C07B 53/00, C07D 307/935

(54) **Optically active alcohols and processes for the preparation thereof**
Optisch-aktive Alkohole und Verfahren zu derer Herstellung
Alcools optiquements actifs et procédé pour leur préparation

(30) Priority: 21.09.1999 JP 26757399
(43) Date of publication of application: 28.03.2001
(73) Proprietor: CHISSO CORPORATION, Osaka-shi Osaka (JP)
(72) Inventor: Ogasawara, Kunio, Sendai-shi, Miyagi-ken (JP)
(74) Representative: Gille Hrabal Struck Neidlein Prop Roos

(56) References cited:
- GB-A- 1 561 314
- PATENT ABSTRACTS OF JAPAN vol. 012, no. 372 (C-533), 5 October 1988 (1988-10-05) & JP 63 122647 A (NISSAN CHEM IND LTD), 26 May 1988 (1988-05-26)
- TAKANO S ET AL: "AN EFFICIENT SYNTHESIS OF CIS-OXABICYCLO[3.3.0]OCT-6-EN-3-ONE" HETEROCYCLES, vol. 16, no. 4, 1981, pages 605-608, XP000571014 ISSN: 0385-5414
- SUGAHARA, T. ET AL.: "A new Chiral Route to Optically Pure 3-Oxodicyclopentadiene : A Versatile Chiral Cyclopentadienone Synthon, via Contra-Steric Diels-Alder Reaction" SYNTHESIS, September 1996 (1996-09), pages 1101-1108, XP000941631
- NAKASHIMA, H. ET AL.: "Lipase-mediated Resolution of cis-4-Cumyloxy-2-cyclopenten-1-ol and its Utilzation for Enantioconvergent Preparation of (-)-Oxabicyclo[3.3.0]-oct-6-en-3-one" SYNLETT, no. 11, 27 October 1999 (1999-10-27), pages 1754-1756, XP000941518
- NAKASHIMA, H. ET AL.: "Chiral Preparation of Polyoxygenated Cyclopentanoids" SYNTHESIS, no. 6, 2000, pages 817-823, XP000941517

## Description

The present invention relates to optically active alcohols and the analogues thereof, which are useful as an intermediate for the synthesis of biologically active compounds such as prostaglandins. More specifically, the invention relates to optically pure cis-4-cumyloxy-2-cyclopenten-1-ol, its analogues and the processes of the preparation thereof. The invention also relates to the use of the optically active alcohols for the synthesis of (-)-oxabicyclo[3.3.0]oct-6-en-3-one.

### BACKGROUND OF THE INVENTION

Recently, chiral alcohols with a plurality of hydroxy groups, which have wide and versatile utility serving as both enantiomers in the synthesis of biologically and/or physiologically active compounds, have become a focus of interest. In particular, mono-protected cis-1,4-dihydroxy-2-cyclopentenes are used as important building blocks for the synthesis of prostaglandins and a variety of biologically active compounds.

Stork et al. (J. Am. Chem. Soc., 97, 6260 (1975)) describe the synthesis of prostaglandins from racemic 4-cumyloxy-2-cyclopenten-1-ol. Stork's process starting from a mixture of cis- and trans-4-cumyloxy-2-cyclopenten-1-ol, however, forms an intended prostaglandin (PGF_{2α}) together with an epimer (15-epi PGF_{2α}). Thus, the Stork's process has several problems that it requires an operation for the isolation of a compound having the intended structural coordination, i.e. PGF_{2α}, from the epimer and that yield of the intended compound is remarkably low.

Accordingly, there has been a demand for an optically pure cis-4-cumyloxy-2-cyclopenten-1-ol and the analogues thereof. However, there is no report on the synthesis of these compounds.

Takano S. et al. report in Heterocycles 16, 605 (1981) the method for efficiently synthesising a racemic compound, 1,4-dihydroxy-2-cyclopentenes, one hydroxyl group of which is protected by t-butyl group, starting from cyclopentadiene. Further, Sugahara T. et al. report in Synthesis, 1101 (1996) that the above compound is optically resolved by the transesterification with vinyl acetate in the presence of a hydrolase in benzene to afford the chiral acetate and the chiral alcohol in excellent yields. However, the process has problems in that it uses benzene as a solvent, which is environmentally unacceptable, and that the enantiomeric purities of the products are not necessarily sufficient.

### SUMMARY OF THE INVENTION

The present invention provides an improved process for preparing an optically pure cis-4-cumyloxy-2-cyclopenten-1-ol, which comprises subjecting a racemic cis-4-cumyloxy-2-cyclopenten-1-ol to the transesterification or the esterification by treating with a carboxylic acid or the ester thereof in the presence of a hydrolase.

The invention also provides a novel process for efficiently synthesising an optically active lactone, (-)-2-oxabicyclo[3.3.0]-oct-6-en-3-one, which is useful as an intermediate for synthesising prostaglandins, utilizing the optically active alcohols as obtained above.

### DETAILED DESCRIPTION OF THE INVENTION

Accordingly, the present invention provides an optically active compound, i.e., (+)-cis-4-cumyloxy-2-cyclopenten-1-ol, represented by formula (1)

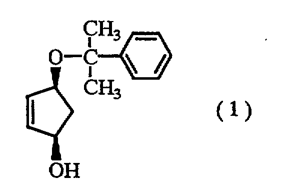

The invention also provides an optically active compound, i.e., (-)-cis-1-acyloxy-4-cumyloxy-2-cyclopentene, represented by formula (2) 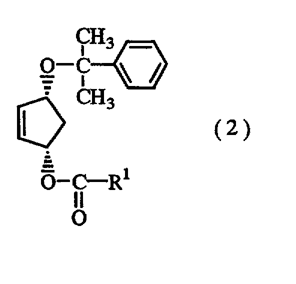 wherein R¹ is a hydrogen atom or a straight or branched C₁-C₁₀ alkyl group, optionally any hydrogen atom in the alkyl group being substituted by a halogen atom.

Examples of the alkyl group for R¹ in formula (2) include, but not limited to, methyl, ethyl, n-propyl, isopropyl, n-butyl, isobutyl, sec-butyl, t-butyl, pentyl, hexyl, chloromethyl, dichloromethyl and trichloromethyl. Preferably, R¹ is methyl. Thus, a preferable compound of formula (2) is (-)-cis-1-acetoxy-4-cumyloxy-2-cyclopentene.

The present invention also provides an optically active compound, i.e., (-)-cis-4-cumyloxy-2-cyclopenten-1-ol, represented by formula (3)

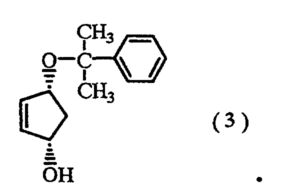

The present invention also provides a process for preparing optically active compounds of formula (1) 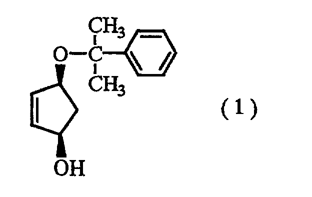 and formula (2) 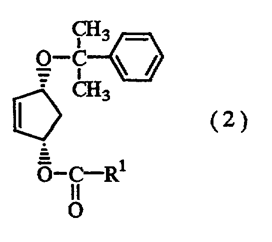 wherein R¹ is a hydrogen atom or a straight or branched C₁-C₁₀ alkyl group, optionally any hydrogen atom in the alkyl group being substituted by a halogen atom, which comprises treating
(±)-cis-4-cumyloxy-2-cyclopenten-1-ol of formula (4) 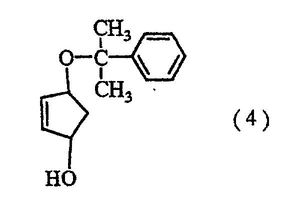 with a hydrolase in the presence of carboxylic acid or its ester of formula (5) 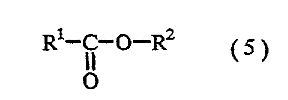 wherein R¹ is as defined above and R² is a hydrogen atom, a straight or branched C₁-C₁₀ alkyl or a straight or branched C₂-C₁₀ alkenyl group, optionally any hydrogen atom in the alkyl or alkenyl group being substituted by a halogen atom.

In the process for preparing the optically active alcohols and the analogues thereof according to the present invention, examples of R² in formula (5) include, but not limited to, a hydrogen atom, methyl, ethyl, propyl, chloromethyl, dichloromethyl, trichloromethyl, vinyl and 2,2,2-trichloroethyl. Preferably, R² is vinyl and thus a preferable compound of formula (5) is vinyl acetate.

Further, the present invention provides a process for preparing an (-)-2-oxabicyclo[3.3.0]oct-6-en-3-one of formula (6) 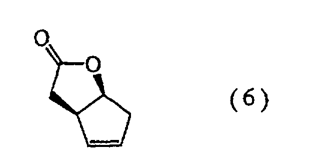 which comprises the steps of;
(a1) heating a compound of formula (1) 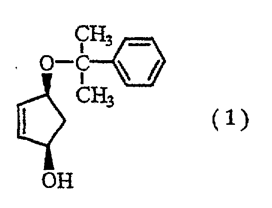 in the presence of dimethylacetamide dimethyl acetal to form a compound of formula (a-1) 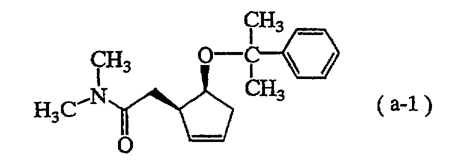 and
(a2) treating the compound (a-1) with an acid in a solvent to form the compound of formula (6).

The invention also provides a process for preparing an (-)-2-oxabicyclo[3.3.0]oct-6-en-3-one of formula (6) 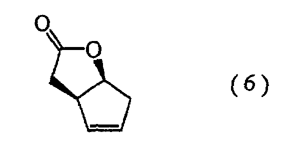 which comprises steps of;
(b1) subjecting a compound of formula (2) 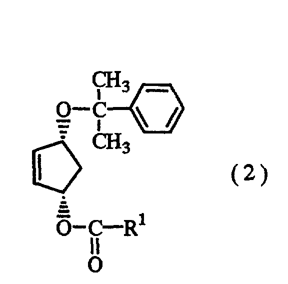 wherein R¹ is as defined above to alcoholysis to form a compound of formula (3) 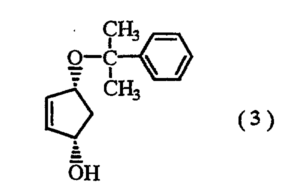
(b2) protecting a hydroxyl group of the compound (3) by a suitable protecting group to form a compound of formula (b-2) 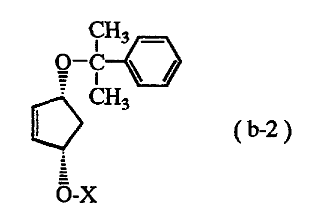 wherein X is a protecting group,
(b3) treating the compound (b-2) with an alkaline metal in ammonia to form a compound of formula (b-3) 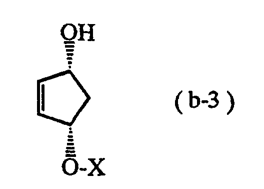 wherein X is as defined above,
(b4) heating the compound (b-3) in the presence of dimethylacetamide dimethyl acetal to form a compound of formula (b-4) 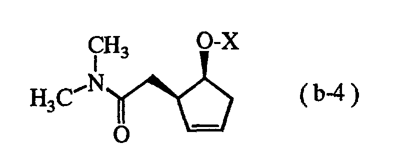 wherein X is as defined above, and
(b5) treating the compound (b-4) with an acid in a solvent to form the compound of formula (6).

In the process of the invention, a suitable protecting group for X can include any group for the protection of hydroxyl groups known in the art of organic synthesis. Examples of such protecting group include, but not limited to, trimethylsilyl, triethylsilyl, t-butyldimethylsilyl (TBS), t-butyldiphenylsilyl, methoxymethyl, methoxyethoxymethyl, t-butyl, benzyl, triphenylmethyl, isopropyldimethylsilyl, tribenzylsilyl and triisopropylsilyl. Preferably, the protecting group is t-butyldimethylsilyl (TBS). Alkaline metals which may be used in the step (b3) include, but not limited to, lithium, sodium and potassium. Preferably, the alkaline metal is sodium.

### PREFERRED EMBODIMENTS OF THE INVENTION

The processes of the present invention can be performed as described below.

### 1. Preparation of optically active alcohols and analogues thereof

The compound of formula (4), a racemic cis-4-cumyloxy-2-cyclopenten-1-ol which is used as the starting material in the present process, can be prepared by affording (±)-cis-4-cumyloxy-2-cyclopentenone from dicyclopetadiene and reducing the resulting compound with sodium borohydride in the presence of cerium(III) chloride heptahydrate in accordance with a method known in the art (e.g. Stork et al., J. Am. Chem. Soc., 97, 6260 (1975)).

The optically active alcohol and its carboxylate according to the invention, i.e. (+)-cis-4-cumyloxy-2-cyclopenten-1-ol and (-)-cis-1-acyloxy-4-cumyloxy-2-cyclopentene, can be prepared by subjecting (±)-cis-4-cumyloxy-2-cyclopenten-1-ol as obtained above to transesterification with carboxylic acid or its ester using a hydrolase in the presence of a solvent to optically resolve the racemic compound.

The transesterification can be performed at a temperature in the range of 0°C to 100°C, preferably 20°C to 40°C, e.g. room temperature. The reaction time is in the range of 1 to 1000 hours, preferably 2 to 10 hours.

The hydrolases which can be used herein, include, but are not limited to, lipase, esterase, protease, and lipoprotein lipase. The hydrolases may be any of animal, plant and fungus origins and those originated from *pseudomonas*, *candida* and *pancreatin* are preferable. The hydrolase may be commercially available immobilized products or dried extracts.

The solvents which can be used in the transesterification concretely include, but are not limited to, hydrocarbon solvents such as hexane, heptane, cyclohexane and toluene; alcohols such as methanol, ethanol, propanol; ketones such as acetone and methylethyl ketone; ethers such as diethyl ether, dimethyl ether, diisopropyl ether, diphenyl ether, methyl butyl ether, tetrahydrofuran and dioxane; and non-protonic polar solvents such as dimethylformamide, dimethylacetamide and dimethylsulfoxide. Preferably, the solvent is t-butyl methyl ether.

The process for preparing the optically active compound of formula (1) and its carboxylate of formula (2), i.e. (+)-cis-4-cumyloxy-2-cyclopenten-1-ol and (-)-cis-1-acyloxy-4-cumyloxy-2-cyclopentene, are shown in the following scheme A.

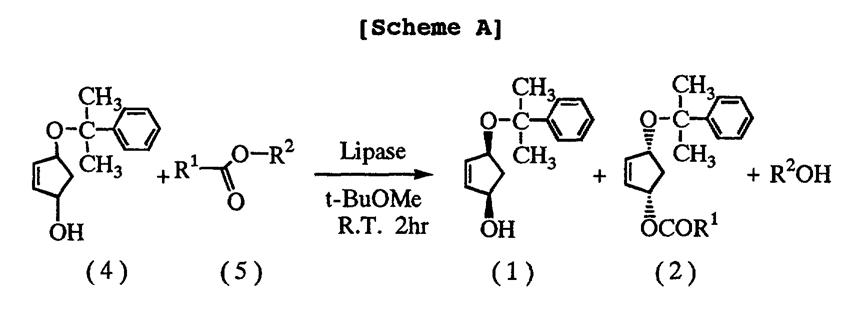

The optically active compounds of formulas (1) and (2) can be prepared by subjecting the racemic compound (4) to the transesterification by treating with immobilized lipase (Lipase PS originated from *pseudomonas* sp., manufactured by Amano Pharm. Co., Ltd.) and carboxylic acid ester (5) in t-butyl methyl ether and then isolating the products by silica gel column chromatography.

### 2. Preparation of (-)-2-oxabicylclo[3.3.0]oct-6-en-3-one

A process for preparing the title compound in which (+)-cis-4-cumyloxy-2-cyclopenten-1-ol is used as a starting material is illustrated below, in order of steps (a1) to (a2).

### Step (a1)

The alcohol of formula (1) is heated with dimethylacetamide dimethyl acetal in a refluxing organic solvent to afford 3R,4S-N,N-dimethyl-4-cumyloxycyclopentenyl-3-acetamide. Examples of solvents which can be used in the step include hydrocarbon solvents such as hexane, heptane, cyclohexane and toluene; alcohols such as methanol, ethanol and propanol; ketones such as acetone and methylethyl ketone; ethers such as diethyl ether, dimethyl ether, diisopropyl ether, diphenyl ether, methyl buthyl ether, tetrahydrofuran and dioxane; and non-protonic polar solvents such as dimethylformamide, dimethylacetamide and dimethylsulfoxide. Preferably, the solvent is diphenyl ether. The reaction temperature is in the range of 200°C to 300°C, preferably 250°C to 280°C, e.g. 280°C. The reaction time varies dependent on the temperature. For example, at the reaction temperature of 280°C, the reaction time is preferably within 30 minutes.

### Step (a2)

The compound from the step (a1) is treated with an acid in the solvent to afford the intended compound. The reaction temperature is in the range of 0°C to 30°C, e.g. room temperature. The reaction time varies dependent on the temperature. At room temperature, for example, the reaction time is in the range of 1 to 24 hours, preferably 12 hours. The solvents which can be used in this step are solvents examplified for the step (1). Preferably, the solvent is dioxane. Examples of the acid which may be used in this step are, but not limited to, a diluted inorganic or organic acid, e.g. hydrochloric acid, sulfuric acid, p-toluenesulphonic acid and pyridinium p-tolunesulfonate. Preferably, the acid is diluted hydrochloric acid or diluted sulfuric acid, e.g. 10% hydrochloric acid. Accordingly, the step is preferably carried out in a mixture of 10% hydrochloric acid and dioxane at the ratio 1:1.

The steps as mentioned above are shown in the following scheme B.

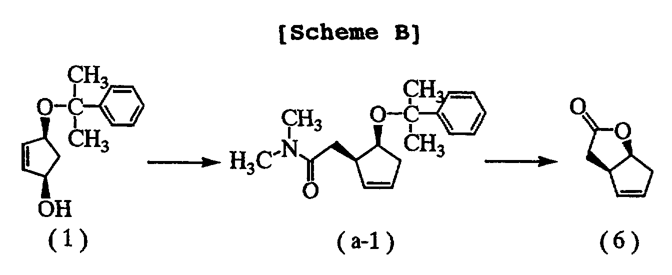

In a preferable embodiment of the present invention, the optically active compound (1) is subjected to the Ecshenmoser reaction by heating in the presence of dimethylacetamide dimethyl acetal in a refluxing solvent such as diphenyl ether to afford 3R,4S-N,N-dimethyl-4-cumyloxycyclopentenyl-3-acetamide of formula (a-1). Subsequently, the resulting compound (a-1) is subjected to the hydrolysis of amide, the elimination of o-cumyl group and the following intramolecular cyclization by treating with 10% hydrochloric acid in dioxane to afford (-)-2-oxabicyclo[3.3.0]oct-6-en-3-one of formula (6).

A process for preparing the lactone of formula (6) in which (-)-cis-1-acyloxy-4-cumyloxy-2-cyclopentene is used as a starting material is illustrated below, in order of steps (b1) to (b5).

### Step (b1)

The compound of formula (2) is subjected to an alcoholysis by exposing to an alkaline alcohol to afford (-)-cis-4-cumyloxy-2-cyclopenten-1-ol of formula (3). Examples of the alkali which may be used in this step, include, but not limited to, sodium hydroxide, potassium hydroxide, sodium carbonate, potassium carbonate and the like. Examples of the alcohol include, but are not limited to, methanol, ethanol, n-propanol, isopropanol, n-butanol and the like.

### Step (b2)

A hydroxyl group of the compound (3) is protected with a protecting group as mentioned above, preferably t-butyldimethylsilyl (TBS) group according to a suitable method known in the art for the protection of hydroxyl groups.

### Step (b3)

The compound (b-2) in which the hydroxyl group is protected is subjected to the Birch reaction by treating with an alkaline metal, preferably sodium in ammonia to afford the compound (b-3). In this reaction, the protecting group is never eliminated.

### Step (b4)

The compound (b-3) is heated together with dimethylacetamide dimethyl acetal in a refluxing organic solvent in the same manner as step (a1) to afford the compound of formula (b-4). The solvent, the reaction temperature and the reaction time employed in this step are similar to those of the above step (a1).

### Step (b5)

The compound (b-4) is stirred with the acid in a solvent in the same manner as step (a2) to afford the intended compound (6), i.e. (-)-2-oxabicyclo [3.3.0]oct-6-en-3-one. The solvent, the reaction temperature and the reaction time employed in this step are similar to those of the above step (a2).

These steps are shown in the following scheme C.

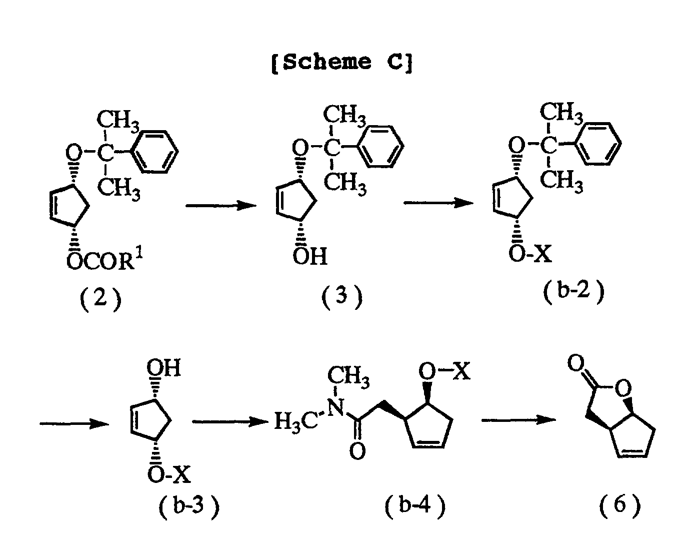

In a preferable embodiment of the present invention, the compound (2) is subjected to the hydrolysis of the ester group by treating with alkaline in methanol, i.e. the Eschenmoser reaction to afford the mono-hydroxyl compound (3), (-)-cis-4-cumyloxy-2-cyclopenten-1-ol (step b1). The hydroxyl group is protected with t-butyldimethylsilyl group (step b2) and then the compound (b-2) is treated with sodium/ammonia to give the mono-hydroxyl compound of formula (b-3) (step b3). The resulting compound (b-3) is processed in the same manner as step (a1) to afford the amide (b-4) (step b4). The amide (b-4) is subjected to the hydrolysis and the intramolecular cyclization by treating with a diluted hydrochloric acid to afford the compound (6) (step b5).

The invention is further illustrated by the following Examples. These examples are not to be construed as limiting the scope of the invention.

### EXAMPLES

### Example 1

### Preparation of (+)-cis-4-cumyloxy-2-cyclopenten-1-ol and (-)-cis-1-acetoxy-4-cumyloxy-2-cyclopentene

A solution of (±)-cis-4-cumyloxy-2-cyclopenten-1-ol (1.071g, 4.91 mmol) and 2.5 ml of vinyl acetate was stirred with Lipase PS immobilized on Celite (1.0 g) at room temperature for 2 hours. After the reaction, the reaction mixture was subjected to filtration to remove the enzyme and then the solvent was distilled off under reduced pressure. The residue was purified on silica gel column (n-hexane/ethyl acetate = 1/4 to 1/2) to afford (+)-cis-4-cumyloxy-2-cyclopenten-1-ol (535 mg, 50% yield), with the following data:
[α]_{D}³¹ +27.52° (c 1.11, CHCl₃)
IR ν max (neat) cm⁻¹: 3410
¹H-NMR (300MHz, CDCl₃) δ: 1.56 (3H, s), 1.58 (3H, s), 1.61 (1H, ddd, *J* = 13.8, 7.2, 7.2 Hz), 2.57 (1H, ddd, *J* = 13.8, 4.8, 4.8 Hz), 4.05-4.12 (1H, m), 4.43-4.52 (1H, m), 5.83 (1H, ddd, *J* = 5.7, 1.5, 1.5 Hz), 5.91 (1H, ddd, *J* = 5.7, 1.5, 1.5 Hz), 7.23-7.48 (5H, m)
¹³C-NMR (75MHz, CDCl₃) δ: 28.64, 29.60, 43.94, 74.98, 76.20, 77.84, 126.18, 127.17, 128.32, 136.07, 136.17, 146.82
MS m/z: 203 (M + -Me)
Exact mass calcd. for C₁₃H₁₅O₂ (M + -Me): 203.1072 Found: 203.1054
Anal. calcd. for C₁₄H₁₈O₂ (M +): C, 77.03, H, 8.31. Found: C, 76.59, H, 8.04;
and
(-)-cis-1-acetoxy-4-cumyloxy-2-cyclopentene (543 mg, 43% yield), with the following data:
[α]_{D}²⁹ -62.02° (c 0.98, CHCl₃)
IR ν max (neat) cm⁻¹: 1738
¹H-NMR (300MHz, CDCl₃) δ: 1.56(3H,s), 1.57 (3H, s), 1.71 (1H, ddd, *J* = 14.1, 4.8, 4.8 Hz), 2.04 (3H, s), 2.66 (1H, ddd, *J* = 14.1, 7.5, 7.5 Hz), 4.00-4.17(1H, m), 5.32-5.38(1H, m), 5.86(1H, ddd, *J* = 6.0, 1.5, 1.5 Hz), 5.94(1H, ddd, *J* = 6.0, 1.5, 1.5 Hz), 7.23-7.48 (5H, m)
¹³C-NMR (75MHz, CDCl₃) δ: 21.09, 28.61, 29.49, 40.36, 75.92, 76.96, 77.78, 126.12, 127.17, 128.31, 131.65, 138.15, 146.76, 171.06
MSm/z: 260(M+)
Exact mass calcd. for C₁₆H₂₀O₃ (M+): 260.1412 Found: 260.1401
Anal. calcd. for C₁₆H₂₀O₃ (M+): C, 73.82, H, 7.74 Found: C, 73.56, H, 7.79.

Enantiomeric purities of the optically active compounds as obtained above were determined by HPLC (isopropanol:hexane = 1:200 v/v) using a chiral column (CHIRALCEL OD manufactured by Daicel Co. Ltd.), by which both compounds were found >99% ee.

### Example 2

### Preparation of (-)-2-oxabicyclo[3.3.0]oct-6-en-3-one using (+)-cis-4-cumyloxy-2-cyclopenten-1-ol as a starting material

A mixture of (+)-cis-4-cumyloxy-2-cyclopenten-1-ol (2.20 g, 10.08 mmol) and dimethylacetamide dimethyl acetal (3.67 ml, 2.51 mmol) in diphenyl ether (20 ml) was refluxed at 280°C for a period of 30 minutes to afford 3R,4S-N,N-dimethyl-4-cumyloxycyclopentyl-3-acetamide, [α]²⁸_{D} -140.57 (c 1.08, CHCl₃), in 84% yield. The resulting compound then was stirred in a mixture of 10% hydrochloric acid and dioxane (1:1) at room temperature for a period of 12 hours to afford (-)-2-oxabicyclo[3.3.0]oct-6-en-3-one, which is useful as an intermediate for prostaglandins, in 87% yield, with the following data:
mp 43-44°C
[α]_{D}²⁹-103.21° (c 1.02, MeOH)
IR ν max (neat) cm⁻¹: 1772
¹H-NMR (300MHz, CDCl₃) δ: 2.37 (1H, dd, *J* = 18.0, 1.8 Hz), 2.62-2.67 (2H, m), 2.72 (1H, dd, *J* = 19.0, 9.6 Hz), 3.43-3.50 (1H, m), 5.08 (1H, ddd, *J* = 5.4, 5.4, 1.5 Hz), 5.53 (1H, ddd, *J* = 5.7, 4.2, 1.8 Hz), 5.74 (1H, ddd, *J* = 5.7, 4.5, 2.1 Hz)
¹³C-NMR (75MHz, CDCl₃) δ: 33.03, 39.29, 45.36, 82.91, 129.62, 131.26, 176.78
MS m/z: 124 (M +)
Exact mass calcd. for C₇H₈O₂ (M+): 124.0524
Found: 124.0486.

### Example 3

### Preparation of (-)-2-oxabicyclo[3.3.0]oct-6-en-3-one using (-)-cis-1-acetoxy-4-cumyloxy-2-cyclopentene as a starting material

The acetate, (-)-cis-1-acetoxy-4-cumyloxy-2-cyclopentene (10.1 g, 38.8 mmol) was exposed to alkaline methanol (K₂CO₃/MeOH) to afford (-)-cis-4-cumyloxy-2-cyclopenten-1-ol, with the following data:
[α]_{D}³¹ -27.43 (c1.01,CHCl₃)
IR ν max (neat) cm⁻¹: 3410
¹H-NMR (300MHz, CDCl₃) δ: 1.56 (3H, s), 1.58 (3H, s), 1.61 (1H, ddd, *J* = 13.8, 7.2, 7.2 Hz), 2.57 (1H, ddd, *J* = 13.8, 4.8, 4.8 Hz), 4.05-4.12 (1H, m), 4.43-4.52 (1H, m), 5.83 (1H, ddd, *J* = 5.7, 1.5, 1.5 Hz), 5.91 (1H, ddd, *J* = 5.7, 1.5, 1.5 Hz), 7.23-7.48 (5H, m)
¹³C-NMR (75MHz, CDCl₃) δ: 28.64, 29.60, 43.94, 74.98, 76.20, 77.84, 126.18, 127.17, 128.32, 136.07, 136.17, 146.82
MS m/z: 203 (M + -Me)
Exact mass calcd. for C₁₃H₁₅O₂ (M + -Me): 203.1072 Found: 203.1054
Anal. calcd. for C₁₄H₁₈O₂ (M + ): C, 77.03, H, 8.31 Found: C, 76.59, H, 8.04.

The hydroxyl group of the resulting compound was transformed into the t-butyldimethylsilyl (TBS) ether under the standard condition (t-butyldimethylsilyl chloride and imidazole in DMF, at 0°C for 180 minutes) to afford (-)-cis-1-(t-butyldimethyl silyloxy)-4-cumyloxy-2-cyclopentene in 97% yield. The compound obtained was then treated with sodium in ammonia and tetrahydrofuran (THF) to afford the mono-hydroxyl compound, [α]_{D}³⁰ -24.53(1.59, CH₂Cl₂) (b-3), in 83% yield. The mono-hydroxyl compound was refluxed with dimethylacetamide dimethyl acetal in diphenyl ether (20 ml) at 280°C for a period of 30 minutes under the same condition as Example 2 to afford 3R,4S-N,N-dimethyl-4-(t-butyldimethyl silyloxy)-cyclopentenyl-3-acetamide (b-4), [α]²⁷_{D} -56.55(c 1.01, CHCl₃), in 77% yield. The compound (b-4) was then stirred in a mixture of 10% hydrochloric acid and dioxane (1:1) at room temperature for a period of 12 hours to afford (-)-2-oxabicyclo[3.3.0]oct-6-en-3-one in 87% yield, with the following data:
mp 43-44°C
[α]_{D}²⁹-103.21° (c 1.02, MeOH)
IR ν max (neat) cm⁻¹: 1772
¹H-NMR (300MHz, CDCl₃) δ: 2.37 (1H, dd, *J* = 18.0, 1.8 Hz), 2.62-2.67 (2H, m), 2.72 (1H, dd, *J* = 19.0, 9.6 Hz), 3.43-3.50 (1H, m), 5.08 (1H, ddd, *J* = 5.4, 5.4, 1.5 Hz), 5.53 (1H, ddd, *J* = 5.7, 4.2, 1.8 Hz), 5.74 (1H, ddd, *J* = 5.7, 4.5, 2.1 Hz)
¹³ C-NMR (75MHz, CDCl₃) δ: 33.03, 39.29, 45.36, 82.91, 129.62, 131.26, 176.78
MS m/z: 124 (M +)
Exact mass calcd. for C₇H₈O₂ (M +): 124.0524
Found: 124.0486.

### INDUSTRIAL APPLICABILITY

The optically pure cis-4-cumyloxy-2-cyclopenten-1-ol and the analogues thereof provided by the present invention are useful intermediates for the synthesis of biologically and/or physiologically active compounds, and especially they are advantageously used as a starting material for the synthesis of prostaglandins. The present invention can also provide a shortened and simplified process for preparing an (-)-2-oxabicyclo[3.3.0]oct-6-en-3-one which is useful as an intermediate for prostaglandins in higher yield in comparison with the prior art methods starting from a racemic compound or a cis/trans mixture. Accordingly, the present invention can provide a novel process for preparing the optically active lactone.

## Claims

1. An optically active compound of formula (1).

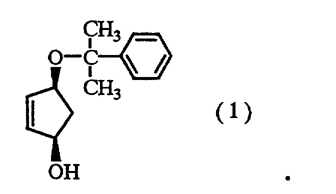

2. An optically active compound of formula (2) 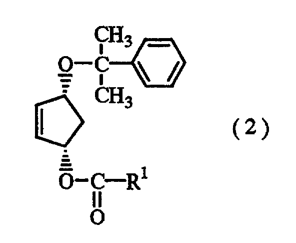 wherein R¹ is a hydrogen atom or a straight or branched C₁-C₁₀ alkyl group, optionally any hydrogen atom in the alkyl group being substituted by a halogen atom.

3. The compound of claim 2, wherein R¹ is the C₁-C₆ alkyl.

4. The compound of claim 3, wherein R¹ is methyl.

5. An optically active compound of formula (3)

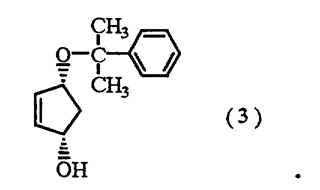

6. A process for preparing optically active compounds of formula (1) 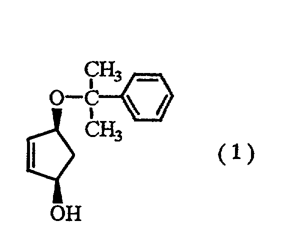 and formula (2) 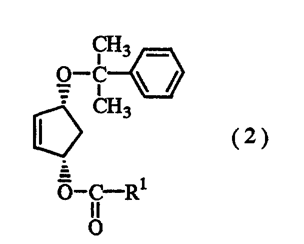 wherein R¹ is a hydrogen atom or a straight or branched C₁-C₁₀ alkyl group, optionally any hydrogen atom in the alkyl group being substituted by a halogen atom, which comprises treating
(±)-cis-4-cumyloxy-2-cyclopenten-1-ol of formula (4) 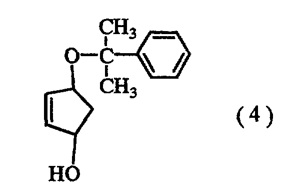 with a hydrolase in the presence of carboxylic acid or its ester of formula (5) 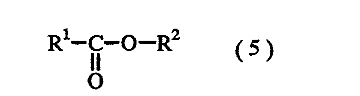 wherein R¹ is as defined above and R² is a hydrogen, a straight or branched C₁-C₁₀ alkyl group or a straight or branched C₂-C₁₀ alkenyl group, optionally any hydrogen atom in the alkyl or alkenyl group being substituted by a halogen atom.

7. The process of claim 6, wherein the hydrolase is a lipase.

8. The process of claim 6 or 7, wherein R¹ is the C₁-C₆ alkyl and R² is the C₂-C₆ alkenyl.

9. The process of claim 8, wherein R¹ is methyl and R² is vinyl.

10. A process for preparing an (-)-2-oxabicyclo[3.3.0]-oct-6-en-3-one represented by formula (6) 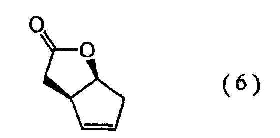 which comprises the steps of;
(a1) heating a compound of formula (1) 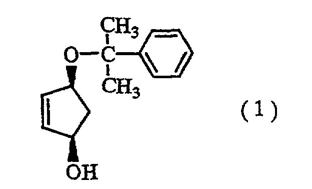 in the presence of dimethylacetamide dimethyl acetal to form a compound of formula (a-1) 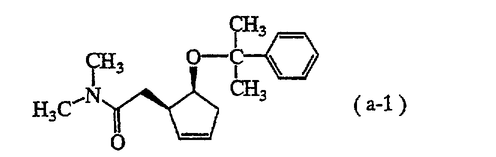 and
(a2) treating the compound (a-1) with an acid in a solvent to form the compound (6).

11. A process for preparing an (-)-2-oxabicyclo [3.3.0]-oct-6-en-3-one of formula (6) 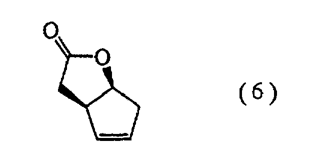 which comprises the steps of;
(b1) subjecting a compound of formula (2) 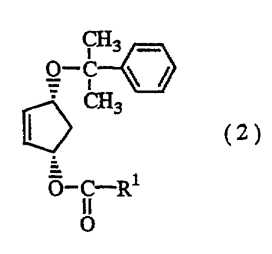 wherein R¹ is a hydrogen atom or a straight or branched C₁-C₁₀ alkyl group, optionally any hydrogen atom in the alkyl group being substituted by a halogen atom, to an alcoholysis to form a compound of formula (3) 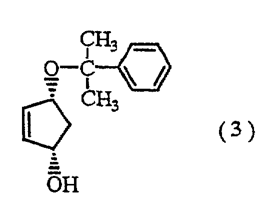
(b2) protecting a hydroxyl group in the compound (3) by a suitable protecting group to form a compound of formula (b-2) 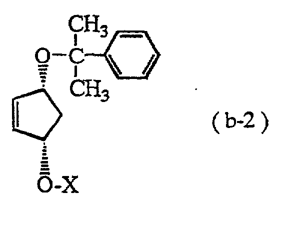 wherein X is a protecting group,
(b3) treating the compound (b-2) with an alkaline metal in ammonia to form a compound of formula (b-3) 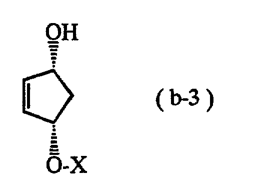 wherein X is as defined above,
(b4) heating the compound (b-3) in the presence of dimethylacetamide dimethyl acetal to form a compound of formula (b-4) 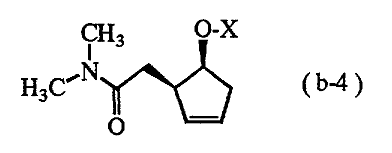 wherein X is as defined above, and
(b5) treating the compound (b-4) with an acid in a solvent to form the compound (6).

12. The process of claim 11, wherein X is t-butyldimethylsilyl group.

## Patentansprüche

1. Optisch aktive Verbindung der Formel (1)

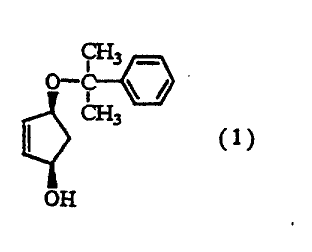

2. Optisch aktive Verbindung der Formel (2) 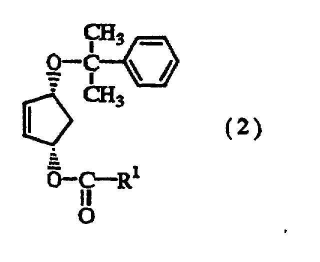 worm R¹ ein Wasserstoffatom oder eine geradkettige oder verzweigte C₁-C₁₀-Alkylgruppe ist, worin wahlweise ein beliebiges Wasserstoffatom in der Alkylgruppe durch ein Halogenatom substituiert sein kann.

3. Verbindung nach Anspruch 2, worin R¹ C₁-C₆-Alkyl ist.

4. Verbindung nach Anspruch 3, worin R¹ Methyl ist.

5. Optisch aktive Verbindung der Formel (3)

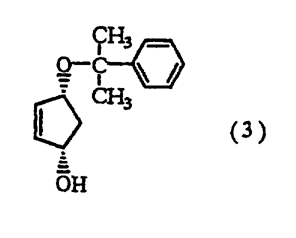

6. Verfahren zur Herstellung optisch aktiver Verbindungen der Formel (1) 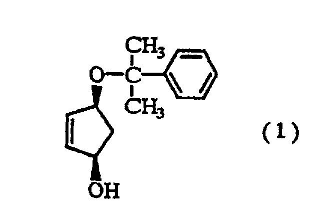 und Formel (2) 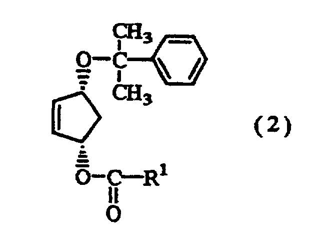 worin R¹ ein Wasserstoffatom oder eine geradkettige oder verzweigte C₁-C₁₀-Alkylgruppe ist, worin wahlweise ein beliebiges Wasserstoffatom in der Alkylgruppe durch ein Halogenatom substituiert sein kann, das umfasst:
Behandlung von (±)-cis-4-Cumyloxy-2-cyclopenten-1-ol der Formel (4) 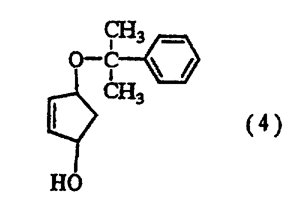 mit einer Hydrolase in Gegenwart einer Carbonsäure oder deren Ester der Formel (5) 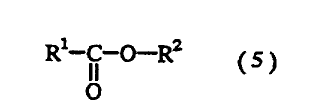 worin R¹ wie oben definiert ist, und R² Wasserstoff, eine geradkettige oder verzweigte C₁-C₁₀-Alkylgruppe oder eine geradkettige oder verzweigte C₂-C₁₀-Alkenylgruppe ist, worin wahlweise ein beliebiges Wasserstoffatom in der Alkyl- oder Alkenylgruppe durch ein Halogenotom substituiert sein kann.

7. Verfahren nach Anspruch 6, worin die Hydrolase eine Lipase ist.

8. Verfahren nach Anspruch 6 oder 7, worin R¹ C₁-C₆-Alkyl und R² C₂-C₆-Alkenyl ist.

9. Verfahren nach Anspruch 8, worin R¹ Methyl und R² Vinyl ist.

10. Verfahren zur Herstellung von (-)-2-Oxobicyclo[3.3.0]-oct-6-en-3-on, dargestellt durch die Formel (6) 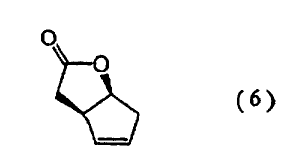 das die Schritte umfasst:
(a1) Erwärmen der Verbindung der Formel (1) 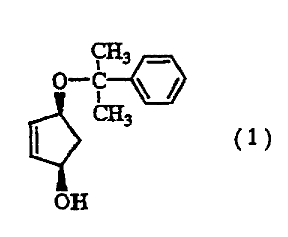 in Gegenwart eines Dimethylacetamiddimethylacetals unter Bildung einer Verbindung der Formel (a-1) 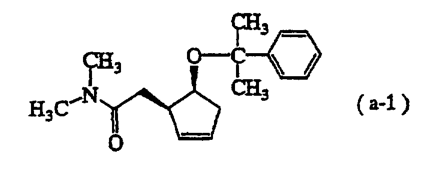 und
(a2) Behandlung der Verbindung (a-1) mit einer Säure in einem Lösungsmittel, um die Verbindung (6) zu bilden.

11. Verfahren zur Herstellung von (-)-2-Oxabicyclo[3.3.0]-oct-6-en-3-on der Formel (6) 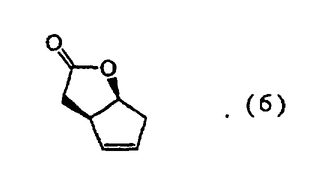 das die Schritte umfasst:
(b1) Unterwerfen einer Verbindung der Formel (2) 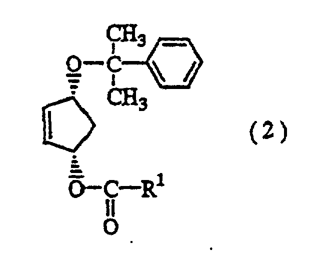 worin R¹ ein Wasserstoffatom oder eine gradkettige oder verzweigte C₁-C₁₀-Alkylgruppe ist, worin wahlweise ein beliebiges Wasserstoffatom in der Alkylgruppe mit einem Halogenatom substituiert sein kann, der Alkoholyse unter Bildung einer Verbindung der Formel (3) 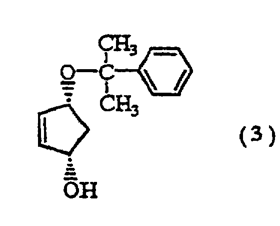
(b2) Schützen der Hydroxylgruppe in der Verbindung (3) durch eine geeignete Schutzgruppe, unter Bildung einer Verbindung der Formel (b-2) 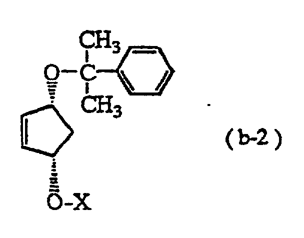 worin X eine Schutzgruppe ist,
(b3) Behandlung der Verbindung (b-2) mit einem Alkalimetall in Ammoniak, unter Bildung einer Verbindung der Formel (b-3) 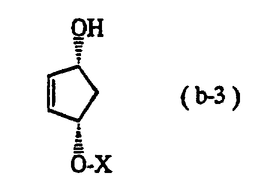 worin X wie oben detiniert ist,
(b4) Erwärmen der Verbindung (b-3) in Gegenwart eines Dimethylacetamiddimethylacetals unter Bildung einer Verbindung der Formel (b-4) 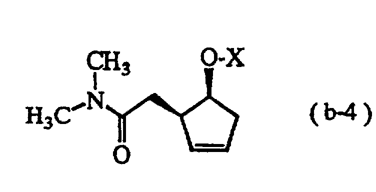 worin X wie oben definiert ist, und
(b5) Behandlung der Verblndung (b-4) mit einer Säure in einem Lösungsmittel, um die Verbindung (6) zu bilden.

12. Verfahren nach Anspruch 11, worin X eine t-Butyldimethylsilyl-Gruppe ist.

## Revendications

1. Composé optiquement actif de formule (1) :

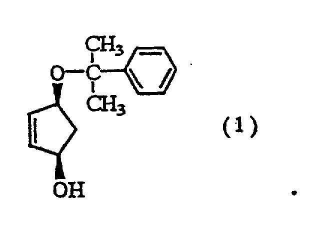

2. Composé optiquement actif de formule (2) : 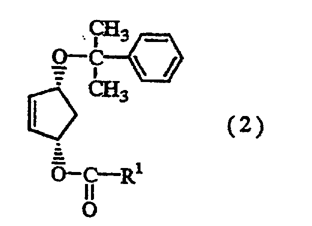 dans laquelle R¹ représente un atome d'hydrogène ou un groupe alkyle en C₁₋₁₀, linéaire ou ramifié, éventuellement n'importe quel atome d'hydrogène dans le groupe alkyle étant remplacé par un atome d'halogène.

3. Composé selon la revendication 2, dans lequel R¹ représente un groupe alkyle en C₁-C₆·

4. Composé selon la revendication 3, dans lequel R¹ représente le groups méthyle.

5. Composé optiquement actif de formule (3) :

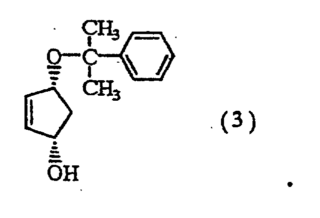

6. Procédé de préparation de composés optiquement actifs de formule (1) 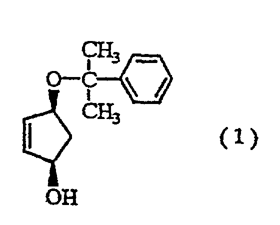 et de formule (2) 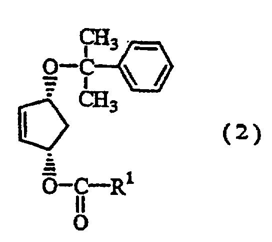 dans laquelle R¹ représente un atome d'hydrogène ou un groupe alkyle en C₁-C₁₀, linéaire ou ramifié, éventuellement n'importe quel atome d'hydrogène dans le groups alkyle étant remplacé par un atome d'halogène ; qui comprend le traitement du (±)-cis-4-cumyloxy-2-cyclopentèn-1-ol de formule (4) 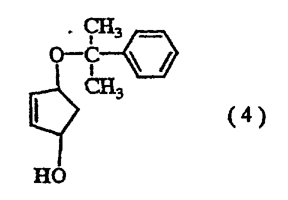 avec une hydrolase en présence d'un acide carboxylique ou de son ester de formule (5) 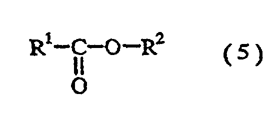 dans laquelle R¹ est tel que défini ci-dessus et R² représente un atome d'hydrogène, un groupe alkyle en C₁₋C₁₀ linéaire ou ramifié ou un groupe alcényle en C₂-C₁₀ linéaire ou ramifié, éventuellement n'importe quel atome d'hydrogène dans le groupe alkyle ou alcényle étant remplacé par un atome d'halogène.

7. Procédé selon la revendication 6, dans lequel l'hydrolase est une lipase.

8. Procédé selon la revendication 6 ou 7, dans lequel R¹ représente un groupe alkyle en C₁-C₆ et R² représente un groupe alcényle en C₂-C₆.

9. Procédé selon la revendication 8, dans lequel R¹ représente le groupe méthyle et R² représente le groupe vinyle.

10. Procédé de préparation d'une (-)-2-oxabicyclo[3.3.0] -oct-6-èn-3-one représentée par la formule (6) 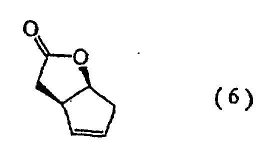 qui comprend les étapes qui consistent à :
(a1) chauffer un composé de formule (1) 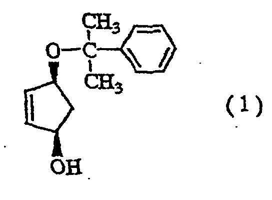 en présence de diméthylacétamide diméthylacétal pour former un composé de formule (a-1) 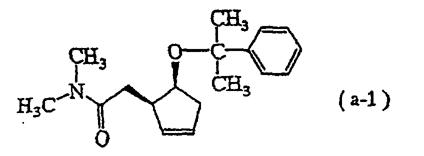 et
(a2) traiter le composé (a-1) avec un acide dans un solvant pour former le composé (6).

11. Procédé de préparation dune (-)-2-oxabicyclo[3.3.0]-oct-6-èn-3-one de formule (6) 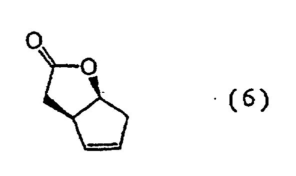 qui comprend les étapes qui consistent à :
(b1) soumettre un composé de formule (2) 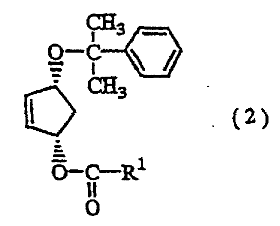 dans laquelle R¹ représente un atome d'hydrogène ou un groupe alkyle en C₁-C₁₀ linéaire ou ramifié, éventuellement n'importe quel atome d'hydrogène dans le groupe alkyle étant remplacé par un atome d'halogène, à une alcoolyse pour former un composé de formule (3) 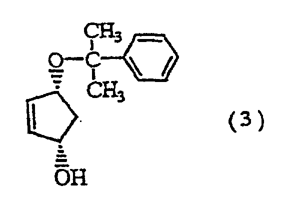
(b2) protéger un groupe hydroxyle dans le composé (3) par un groupe protecteur convenable pour former un composé de formule (b-2) 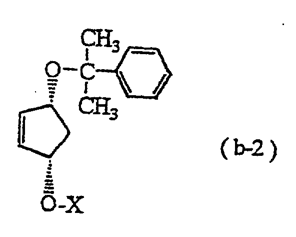 dans laquelle X représente un groupe protecteur,
(b3) traiter le composé (b-2) avec un métal alcalin dans l'ammoniac pour former un composé de formule (b-3) 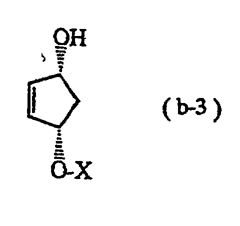 dans laquelle X est tel que défini ci-dessus,
(b4) chauffer le composé (b-3) en présence de diméthylacétamide diméthylacétal pour former un composé de formule (b-4) 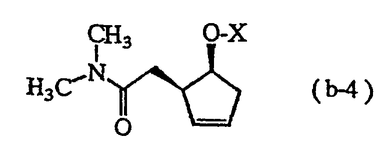 dans laquelle X est tel que défini ci-dessus, et
(b5) traiter le composé (b-4) avec un acide dans un solvant pour former le composé (6).

12. Procédé selon la revendication 11, dans lequel X représente le groupe t-butyldiméthylsilyle.
